(19) Europäisches Patentamt
European Patent Office
Office européen des brevets

(11) EP 4 332 228 A1

(12) **EUROPEAN PATENT APPLICATION**
published in accordance with Art. 153(4) EPC

(43) Date of publication:
**06.03.2024 Bulletin 2024/10**

(21) Application number: **21939426.9**

(22) Date of filing: **30.04.2021**

(51) International Patent Classification (IPC):
***C12N 15/74*** *(2006.01)* ***C12N 9/12*** *(2006.01)*
***C12N 9/16*** *(2006.01)* ***A61K 39/112*** *(2006.01)*
***C12Q 1/02*** *(2006.01)*

(52) Cooperative Patent Classification (CPC):
**A61K 39/0275; C12N 9/12; C12N 9/16;**
**C12N 15/74; C12Q 1/02;** Y02A 50/30

(86) International application number:
**PCT/KR2021/005528**

(87) International publication number:
**WO 2022/231043 (03.11.2022 Gazette 2022/44)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
- **Industry Foundation of Chonnam National University**
  **Gwangju 61186 (KR)**
- **Odysseus Bio Co., Ltd.**
  **Hwasun-gun, Jeollanam-do 58128 (KR)**

(72) Inventors:
- **CHOY, Hyon El**
  **Gwangju 61503 (KR)**
- **KIM, Kwangsoo**
  **Gwangju 61697 (KR)**
- **JEONG, Jae Ho**
  **Gwangju 61967 (KR)**
- **LIM, Dae Jin**
  **Gwangju 61736 (KR)**
- **DUYSAK, Taner**
  **Gwangju 62435 (KR)**
- **TRAN, Quang Thanh**
  **Hwasun-gun Jeollanam-do 58116 (KR)**

(74) Representative: **Plougmann Vingtoft a/s**
**Strandvejen 70**
**2900 Hellerup (DK)**

Remarks:
The complete document including Reference Table(s) and the Sequence Listing(s) can be downloaded from the EPO website

# (54) ATTENUATED SALMONELLA GALLINARUM STRAIN AND USE THEREOF

(57) The present invention relates to an attenuated Salmonella gallinarum strain and use thereof. Particularly, the present invention relates to: a novel attenuated Salmonella strain in which all of a gene encoding guanosine tetraphosphate (ppGpp) synthetase, a gene that induces the function of a type III secretion system (T3SS) (*ssrAB*), and a Gifsy *2prophage* gene are deleted; and a composition for treating or diagnosing tumors by using same.

EP 4 332 228 A1

## Description

Technical Field

The Present invention relates to an attenuated *Salmonella gallinarum* strain and use thereof. Specifically, the present invention relates to a novel attenuated *Salmonella gallinarum* strain in which a gene encoding guanosine tetraphosphate (ppGpp) synthetase, a gene (ssrA, *ssrB,* or ssrAB) inducing the function of a type III secretion system (T3SS), and a Gifsy *2 prophage* gene are all deleted, and to a composition for treating or diagnosing a tumor by using the same.

Background Art

In 2019, a total of 81,203 people died of cancer, accounting for 27.5% of all deaths. Therefore, cancer is considered the most common cause of death, and according to the National Cancer Information Center, the incidence rate has been increasing since 1999, when nationwide cancer incidence statistics began to be collected.

The increase of the incidence rate results from increases in environmental factors, such as increasing environmental pollutants including air pollution, and personal factors, such as intake of high-fat diet due to westernization of diet, drinking, and smoking. Therefore, the development of anti-cancer materials for early prevention and treatment of cancer is becoming more and more important. In addition, refractory adenocarcinomas, for which there is no distinctive therapy, have low incidence rates but high death rates, so the development of medicines therefor is urgently needed. Many compoundbased anticancer agents show non-specificity that the agents act on the whole body in addition to cancer cells, resulting in problems with respect to side effects.

Since bacterial infection was reported to show an anticancer effect, research on the development of anti-tumor bacterial strains has been rapidly increasing. Anti-tumor bacteria researched over the past 20 years are *Bifidobacterium, Clostridium, Lactococcus, Shigella, Vibrio, Listeria, Escherichia, Salmonella,* and the like. However, mechanisms of cancer therapy using these strains have not yet been fully revealed.

Among the various strains mentioned above, existing developed attenuated *Salmonella* strains are distributed not only to cancer cells but also to many normal organs, resulting in a low cancer cell targeting rate, thereby raising issues with respect to side effects by the strains themselves. Especially, immunocompromised patients or the elderly and infirm may develop fetal sepsis caused by even attenuated Salmonella strains, so improvement measures are required even though treatment effects thereof are taken into account. Therefore, there is a need to develop new strains that have significantly higher tumor targeting ability and in vivo stability and can be effectively used for cancer treatment.

Accordingly, the present inventors conducted research to develop attenuated *Salmonella gallinarum* strains that have high in vivo safety, excellent tumor targeting ability, survival rates, and treatment ability, but very low targeting ability and survival rates for normal organs, and thus completed the present invention.

Prior Art Document

Patent Document

(Patent Document 0001) Korean Patent No. 10-2015573

Disclosure of Invention

Technical Problem

The present invention provides a novel attenuated *Salmonella gallinarum* strain in which a gene encoding guanosine tetraphosphate (ppGpp) synthetase, a gene inducing the function of a type III secretion system (T3SS), and a Gifsy *2 prophage* gene are deleted.

Furthermore, the present invention provides a novel attenuated *Salmonella gallinarum* strain in which a gene encoding guanosine tetraphosphate (ppGpp) synthetase, a gene inducing the function of a type III secretion system (T3SS), a Gifsy *2 prophage* gene, and a *glmS* gene are deleted.

Specifically, the present invention provides the *Salmonella gallinarum* SG4044 strain (accession number: KCTC14541BP) or SG4048 strain (accession number: KCTC14542BP).

The present invention provides a composition containing the strain of the present invention for treatment or diagnosis of a tumor.

The present invention provides a method for providing information for tumor diagnosis, the method including: administering the strain of the present invention to a subject; detecting the bioluminescence of the strain; and determining that a tumor is present in the subject when the bioluminescence is detected.

Solution to Problem

The present invention is directed to an attenuated *Salmonella gallinarum* strain in which a gene encoding guanosine tetraphosphate (ppGpp) synthetase, a gene inducing the function of a type III secretion system (T3SS), and a Gifsy *2 prophage* gene are deleted.

Furthermore, the present invention is directed to a novel attenuated *Salmonella gallinarum* strain in which a gene encoding guanosine tetraphosphate (ppGpp) synthetase, a gene inducing the function of a type III secretion system (T3SS), a Gifsy *2 prophage* gene, and a *glmS* gene are deleted.

The present invention is directed to an attenuated *Salmonella gallinarum* strain transformed with a plasmid carrying *glmS* gene operatively linked to a promoter.

The gene encoding guanosine tetraphosphate (ppGpp) synthetase may be *relA* or *spoT*.

The gene inducing the function of a type III secretion system (T3SS) is *ssrA, ssrB,* or *ssrAB* arranged sequentially in the genome.

The present invention is directed to an attenuated *Salmonella gallinarum* strain having deletions of *relA, spoT, ssrAB,* and Gifsy *2 prophage* genes in the *Salmonella gallinarum* SG4021 strain (accession number: KCTC13985BP).

The present invention is directed to an attenuated *Salmonella gallinarum* strain having deletions of *relA, spoT, ssrAB,* Gifsy *2 prophage,* and *glmS* genes in the *Salmonella gallinarum* SG4021 strain (accession number: KCTC13985BP).

In an embodiment, the attenuated *Salmonella gallinarum* strain of the present invention may be the *Salmonella gallinarum* SG4044 strain (accession number: KCTC14541BP).

In an embodiment, the attenuated *Salmonella gallinarum* strain of the present invention may be the *Salmonella gallinarum* SG4048 strain (accession number: KCTC14542BP).

The strain of the present invention may further contain a luminescent gene.

The luminescent gene may be *luxCDABE.*

The present invention is directed to a pharmaceutical composition containing the strain for diagnosis or treatment of a tumor. The tumor may be a solid cancer or adenocarcinoma, and preferably may be colorectal cancer, pancreatic cancer, lung cancer, skin cancer, and breast cancer, but is not limited thereto.

The present invention is directed to a method for providing information for tumor diagnosis, the method including: administering the strain of the present invention to a subject; detecting the bioluminescence of the strain; and determining that a tumor is present in the subject when the bioluminescence is detected.

Advantageous Effects of Invention

The attenuated *Salmonella gallinarum* strain prepared by the lack of guanosine tetraphosphate (ppGpp) producing ability, the function failure of the type III secretion system (T3SS), and the deletion of Gifsy *2 prophage* gene can specifically target a tumor, minimize the damage to normal tissues other than the tumor while having excellent tumor growth inhibiting activity, and be useful in alleviating or treating and imaging the tumor.

Brief Description of Drawings

FIG. 1 shows the results of amino acid requirements of wild-type (SG4021), ppGpp-deleted (SG4023), and ppGpp-, *ssrAB-,* Gifsy 2 *prophage-deleted* (SG4044) *Salmonella gallinarum* strains.

FIG. 2 shows a graph depicting the survival rate of mice according to each dose in the intravenous administration of the wild-type *Salmonella gallinarum* strain (SG4021).

FIG. 3 shows a graph depicting the survival rate of mice according to each dose in the intravenous administration of the ppGpp-, *ssrAB-,* and Gifsy 2 *prophage-deleted Salmonella gallinarum* strain (SG4046).

FIG. 4 shows a graph depicting the survival rate of mice according to each dose in the intravenous administration of the ppGpp-deleted *Salmonella typhimurium* strain (SMR2130).

FIG. 5 shows a graph comparing plasmid stability between the glmS-deleted attenuated *Salmonella gallinarum* strain (SG4050) transformed with *GlmS*$^+$pLux plasmid and the wild-type *Salmonella* strain (SG4021).

FIG. 6 shows images obtained through bioluminescent signal imaging of targeting results for various tumors after the intravenous administration of the attenuated *Salmonella gallinarum* strain (SG4050) transformed with *GlmS*$^+$pLux plasmid.

FIG. 7 shows a graph depicting tumor volumes over time and images depicting changes in transplanted tumors, in CT26 cell-transplanted mice intravenously administered with the ppGpp-, *ssrAB-,* and Gifsy 2 prophage-deleted *Salmonella gallinarum* strain (SG4044) and the ppGpp-deleted *Salmonella typhimurium* strain (SMR2130) (N= 5/group).

FIG. 8 shows a graph depicting the mouse survival rate over time, in CT26 cell-transplanted mice intravenously administered with the ppGpp-, *ssrAB-,* and Gifsy *2 prophage-deleted Salmonella gallinarum* strain (SG4044) and the ppGpp-deleted *Salmonella typhimurium* strain (SMR2130).

FIG. 9 shows graphs depicting the *Salmonella* cell count for each organ, in CT26 cell-transplanted mice intravenously administered with the ppGpp-, *ssrAB-,* and Gifsy *2 prophage-deleted Salmonella gallinarum* strain (SG4046) and the ppGpp-deleted *Salmonella typhimurium* strain (SMR2130) (N= 5/group).

Best Mode for Carrying out the Invention

Hereinafter, embodiments and examples of the present application will be described in detail with reference to the accompanying drawings so that a person skilled in the art to which the present invention pertains can easily carry out the present application. However, the present application can be implemented in various forms and is not limited to the embodiments and examples described herein.

Throughout the specification of the present application, unless otherwise stated, when a certain part "includes" or "comprises" a certain element, it means that the certain part may further include or comprise other elements rather than exclude other elements.

The present invention is directed to an attenuated *Salmonella gallinarum* strain in which a gene encoding guanosine tetraphosphate (ppGpp) synthetase, a gene inducing the function of a type III secretion system (T3SS), and a Gifsy *2 prophage* gene are deleted.

*Salmonella typhimurium* is one of the *Salmonella* strains that are most frequently used in the anticancer targeted therapy research using conventional bacteria, and *Salmonella typhimurium* is necessarily attenuated before use for research considering the pathogenicity thereof (Forbes, N.S. (2010). Engineering the perfect (bacterial) cancer therapy. Nature Reviews Cancer, 10(11), 785-794). The *Salmonella* genus is diverse enough to be classified into approximately 2,500 species according to serological classification, and some of the strains have been reported to possess host-specific pathogens (*Porwollik, S; Boyd, EF; Choy, C; Cheng, P; Florea, L; Proctor, E; McClelland, M (September 2004)).* For example, it has been reported that *Salmonella typhi* and *Salmonella paratyphi* have pathogenic mechanisms that target a human as a host, and *Salmonella typhimurium* causes illness by infecting cattle, pigs, sheep, horses, and rodents, including humans. Whereas, *Salmonella gallinarum* is known to be pathogenic by specifically infecting only birds. Therefore, the attenuated *Salmonella typhimurium* strain of the present invention is the safest for humans and can be effectively used for the diagnosis or treatment of a tumor.

As used herein, the term "attenuated" refers to being modified to lower the pathogenicity of a strain. The attenuation of the strain can prevent cytotoxicity and other side effects that may occur due to the pathogenicity of the strain in normal cells other than tumor cells. Attenuated strains can be constructed through various methods known in the art. For example, the attenuation may be attained by the deletion or destruction of a virulence factor so that a strain can survive in host cells, and may be attained by deletions of genes encoded in Salmonella pathogenicity island (SPI) including *pab, proB'C, nadA, pncB, pmi, rpsL, ompR, htrA, hemA, rfc, poxA, galU, aro, galE, cya, crp, cdt, pur, phoP, phoQ, ssa, guaA, guaB, clpP, clpX, fliD, flgK, flgL, relA, spoA,* and *spoT* genes, and the genes, encoded by Salmonella Pathogenicity Islands (SPI), including *ssaV, sseBCD, ssrAB, sopB, sseF,* and *sseG,* but is not limited thereto.

As used herein, the term "ppGpp" refers to guanosine tetraphosphate and may be used interchangeably with guanosine 5'-diphosphate 3'-diphosphate or guanosine 3',5'-bispyrophosphate. ppGpp, which is an intracellular signaling substance, induces the expression of genes encoded by Salmonella pathogenicity island (SPI) among genes responsible for the toxicity of *Salmonella gallinarum* strains. In addition, the genes encoding guanosine tetraphosphate (ppGpp) synthetase may mean *relA* and *spoT* genes.

The deletions of both *relA and spoT* genes may be attained by the modification of *relA* and *spoT* genes that cause the impairment to the transcription or translation of the genes or the activity of the gene products. Such genetic modifications may include not only the inactivation of a ppGpp synthetase coding sequence (CDS) but also the inactivation of a promoter thereof.

The specific inactivation of only a target gene on the genome of a *Salmonella gallinarum* strain may be attained by the mutation through substitution, insertion, deletion, or a combination thereof on the entire region or at least one partial region of the coding gene. For example, the deletion of a gene or the insertion of a heterogeneous sequence into a gene may result in gene truncation, nonsense mutation, frameshift mutation, missense mutation, and the like. Such the specific gene inactivation may be performed using a method that is usually used in the art. Meanwhile, the deletion of the gene may be performed by various mutagenesis methods that are known in the art. For example, the deletions of *relA* and *spoT* genes may be attained by PCR mutagenesis and cassette mutagenesis.

As used herein, the term "type III secretion system (T3SS)" is regulated by *ssrA* and *ssrB* genes that are sequentially arranged on the genome. The transcriptional regulator *ssrB* is phosphorylated by the membrane protein *ssrA* to have activity. Therefore, the loss of function through the removal of *ssrA* and/or *ssrB* on the genome of *Salmonella*

*gallinarum* may result in the inactivation of the operon and all genes included in the salmonella pathogenicity island 2 (SPI2). After the infection into a host, *Salmonella* distributes the type III secretion system (T3SS) encoded by SPI2 to modify functions of the host cell and reproduce within the host cell.

In the present invention, Gifsy 1 and 2 mean viruses (bacteriophages) targeting bacteria. The present inventors verified according to the genome test results of wild-type *Salmonella gallinarum* that the entire gene sequence encoding Gifsy *2 prophage* is included. The deletion of the gene inserted into the *Salmonella* genome can lower the pathogenicity of *Salmonella.*

The removal of the genome of bacteriophages, which use bacteria as hosts, lowers the pathogenicity to help the attenuation.

Therefore, an attenuated *Salmonella gallinarum* strain in which *relA, spoT,* and *ssrAB* genes and a gene encoding Gifsy *2 prophage* are all deleted may be at least one million times less toxic than wild-type *Salmonella gallinarum,* enabling the effective attenuation of the *Salmonella gallinarum* strain.

The *glmS* gene-deleted *Salmonella gallinarum* strain may be lysed in an animal due to the lack of D-glucosamine (GlcN) or N-acetyl-D-glucosamine (GlcNAc), which is a peptidoglycan synthesis component, and thus the *glmS* gene instead of antibiotic-resistant genes may be used as a selective determinant for the *Salmonella gallinarum* strain.

A balanced-lethal host-vector system may be constructed by transformation of a plasmid carrying *glmS* gene for compensating for the glmS-deleted mutation on the chromosome thereof. The attenuated *Salmonella gallinarum* strain that has been successfully transformed with a plasmid carrying *glmS* gene is capable of biosynthesis of GlcN or GlcNAc and thus can survive even in environments lacking GlcN or GlcNAc, thereby serving as a selective marker.

The plasmid of the present invention may be a plasmid used in the art, and for example, may be one selected from the group consisting of a pcDNA series, pSC101, pGV1106, pACYC177, ColE1, pKT230, pME290, pBR322, pUC8/9, pUC6, pBD9, pHC79, pIJ61, pLAFR1, pHV14, a pGEX series, a pET series, and pUC19, but is not limited thereto.

The plasmid of the present invention may further carry at least one selective marker. The marker is typically a nucleic acid sequence that can be selected by a chemical method, and a gene enabling the differentiation of transformed cells from non-transformed cells may correspond to the marker. For example, the marker may be a gene resistant to an herbicide, such as glyphosate, glufosinate ammonium, or phosphinothricin, or a gene resistant to an antibiotic, such as ampicillin, kanamycin, G418, bleomycin, hygromycin, or chloramphenicol, but is not limited thereto.

The plasmid of the present invention may be prepared by using a gene recombinant technique that is well known in the art, and site-specific DNA cleavage and ligation may be carried out using enzymes that are generally known in the art.

A method for transforming the *Salmonella gallinarum* strain by the introduction of the plasmid of the present invention into the strain may be a method that is generally used to introduce a nucleic acid into cells in the art, and may be performed by selecting a suitable standard technique known in the art. Examples of the technique may be electroporation, calcium phosphate (CaPO4) precipitation, calcium chloride ($CaCl_2$) precipitation, microinjection, polyethylene glycol (PEG) method, DEAE-dextran method, cationic liposome method, and lithium acetate-DMSO method, but are not limited thereto.

As used herein, the term "operably linked" refers to a functional linkage between a gene expression control sequence and another nucleotide sequence. The gene expression control sequence may be at least one selected from the group consisting of a replication origin, a promoter, and a transcription termination sequence (terminator). The transcription termination sequence may be a polyadenylation sequence (pA), and the replication origin may be an f1 replication origin, an SV40 replication origin, a pMB1 replication origin, an adeno replication origin, an AAV replication origin, or a BBV replication origin, but is not limited thereto.

As used herein, the term "promoter" means a region of DNA upstream from the structural gene, and refers to a DNA molecule to which RNA polymerase binds to initiate transcription.

The promoter according to an embodiment of the present invention is one of the transcription control sequences that regulate the transcription initiation of a specific gene, and may be a polynucleotide fragment of about 100 to about 2,500 bp in length. For example, the promoter may be selected from the group consisting of *tac* promoter, *lac* promoter, *lacUV5* promoter, *lpp* promoter, *pLA* promoter, pRA promoter, *rac5* promoter, *amp* promoter, *recA* promoter, *SP6* promoter, trp promoter, *T7* promoter, *pBAD* promoter, *Tet* promoter, trc promoter, *pepT* promoter, *sulA* promoter, *pol11 (dinA)* promoter, *ruv* promoter, *uvrA* promoter, *uvrB* promoter, *uvrD* promoter, *umuDC* promoter, *lexA* promoter, *cea* promoter, *caa* promoter, *recN* promoter, and *pagC* promoter, and synthesized promoters, but is not limited thereto.

As used herein, the term "luminescent gene" refers to a gene encoding a protein enabling in vivo or ex vivo (in vitro) imaging, and examples thereof may include various-derived bioluminescent genes (e.g., luciferase gene), chemiluminescent genes, and fluorescent genes. An imaging device for the luminescence expressed by such genes is known in the art and may be appropriately selected by a person skilled in the art.

The *Salmonella gallinarum* strain transformed with a plasmid containing the luminescent gene of the present invention targets a tumor, enabling visual monitoring of the tumor, thereby increasing the accuracy and effectiveness of tumor treatment. The imaging device that can be used together with the luminescent gene of the present invention may be appropriately selected by a person skilled in the art according to the type of luminescent gene.

The *"luxCDABE"* of the present invention is a DNA fragment containing both a gene (a heterogeneous dimer of *luxA* and *luxB*) encoding luciferase and genes (*luxC, luxD,* and *luxE*) encoding luciferin (tetradecanal) as a substrate of the luciferase. Luciferin as a luminescent substance is activated by ATP to be converted to be activated luciferin in cells, and the activated luciferin becomes oxidized into luciferin by action of luciferase as a luminescent enzyme, converting chemical energy into light energy to emit light.

Tumor cells targeted by the *Salmonella gallinarum* strain transformed with the luminescent gene of the present invention can be detected by the luminescent gene, thereby obtaining information on the presence or absence of a tumor in a subject, the location and size of a tumor in a subject, and the change pattern of a tumor present in a subject over time.

In the present invention, the targeting and anticancer activity of the attenuated *Salmonella gallinarum* strain on colorectal cancer, pancreatic cancer, lung cancer, skin cancer, and breast cancer cell lines were confirmed, and the excellent anticancer effect of the attenuated *Salmonella gallinarum* strain of the present invention was confirmed in a CT26 tumor mouse model with respect to one of adenocarcinomas.

The attenuated *Salmonella gallinarum* strain of the present invention can exhibit targeting and tumor inhibitory activity on various adenocarcinomas or solid cancers, such as colorectal cancer, pancreatic cancer, lung cancer, skin cancer, and breast cancer.

Adenocarcinoma is a type of cancer that occurs in cells constituting, especially, glands, and refers to cancer developed in not only mucous membranes in stomach, intestine, bronchus, uterine, gall bladder, or the like, but also glandular tissues or excretory ducts in prostate, thyroid gland, and pancreas.

Hereinafter, the present invention will be described in detail with reference to examples. However, the following examples are illustrative only and do not limit the scope of the present invention.

**[Example 1]**

**Construction of attenuated *Salmonella gallinarum* strain and evaluation of tumor inhibitory activity thereof**

**1-1. Salmonella strain growth**

*Salmonella spp.* was grown at 37°C in LB media (Difco Laboratories) containing 1% NaCl under vigorous aeration. Solid support media were prepared containing 1.5% granule agar (Difco Laboratories), and antibiotics were purchased from Sigma chemical. If necessary, the antibiotics ampicillin (Amp), kanamycin (Km), and chloramphenicol (Cm) were added at concentrations of 100 pg/ml, 50 pg/ml, and 15 pg/ml, respectively, and N-acetyl-D-glucosamine (GlcNAc) was added at a concentration of 100 mg/ml. The bacteria count in the liquid media was counted using a hemocytometer.

**1-2. Salmonella strain lineages**

As shown in Table 1 below, serotype *Gallinarum* mutants were originated from the clinical isolate SG4021 isolated from the liver of chickens with fowl typhoid bred in a Korean farm, and the SG4021 strain was deposited with the Korean Research Institute of Bioscience and Biotechnology (accession number: KCTC13985BP) on 8 October 2019. The SG4044 and SG4048 strains were deposited with the Korean Research Institute of Bioscience and Biotechnology (accession numbers: KCTC14541BP and KCTC14542BP) on 20 April 2021.

TABLE 1

| Strain | Lineage | Indication | Method |
|---|---|---|---|
| *Salmonella gallinarum* | SG4021 | Wild type-clinical isolate | |
| | SG4022 | *relA::kan*$^R$, | |

(continued)

| Strain | Lineage | Indication | Method |
|---|---|---|---|
| | | *spoT::cm$^R$* | |
| | SG4023 | *ΔrelA, ΔspoT* | |
| | SG4030 | *ΔrelA, ΔspoT, glmS:: kan$^R$* | |
| | SG3005 | *ΔssrAB:: kan$^R$* | |
| | SG4041 (DJ1110) | *ΔrelA, Δspot, ssrAB:: kan$^R$* | P22 (from SG3005) Transduction |
| | SG4042 (TH1001) | Gifsy *2 prophage:: cm$^R$* | Lambda-Red homologous recombination |
| | SG4043 (TH1004) | SG4041, *ΔrelA, Δspot, ssrAB:: kan$^R$,* Gifsy 2 *prophage:: cm$^R$* | P22 (from SG4042) Transduction |
| | SG4044 (TH1005) | SG4043, *ΔrelA, Δspot, ΔssrAB,* ΔGifsy *2 prophage* | Transformation with pCP20 |
| | SG4045 | SG4041, *ΔrelA, Δspot, ΔssrAB* | Transformation with pCP20 |
| | SG4046 | SG4045, *ΔrelA, Δspot, ΔssrAB,* ΔGifsy *2 prophage:: cm$^R$* | P22 (from SG4042) Transduction |
| | SG4047 | SG4044, *ΔrelA, Δspot, ΔssrAB,* ΔGifsy *2 prophage, glmS:: kan$^R$* | P22 (from SG4030) Transduction |
| | SG4048 | SG4047, *ΔrelA, Δspot, ΔssrAB,* ΔGifsy *2 prophage, ΔglmS* | Transformation with pCP20 |
| | SG4049 | SG4044, *ΔrelA, Δspot, ΔssrAB,* ΔGifsy *2 prophage* /*GlmS$^+$_$_P$Lux* | Transformation with *GlmS$^+$_$_P$Lux* |
| | SG4050 | SG4048, *ΔrelA, Δspot, ΔssrAB,* ΔGifsy *2* | Transformation with *GlmS$^+$_$_P$Lux* |
| | | *prophage, ΔglmS*/*GlmS$^+$_$_P$Lux* | |
| *Salmonella typhimurium* | SHJ2037 | *relA::kanR, spoT::cm$^R$* | |
| | SMR2130 | Δ*relA,* Δ*spoT* | |

All the Salmonella strain lineages were constructed according to the methods developed by Datsenko and Wanner (Proceedings of the National Academy of Sciences 97.12 (2000): 6640-6645), and the attenuation of the Salmonella strains was induced by the deletions of ppGpp, *ssrAB,* and Gifsy *2 prophage* (ΔppGpp, Δ*ssrAB,* ΔGifsy *2 prophage*).

The ppGpp-deleted mutant was induced by sequentially introducing *relA::kan$^R$* and spot::*cm$^R$* into the genome of SG4021. The ssrAB-deleted mutant was induced by introducing *ssrAB::kan$^R$* into the genome of SG4021 (SG3005) . For the construction of ppGpp- and *ssrAB*-deleted mutants, the antibiotic-resistant genes introduced for ppGpp preparation were first inactivated *(kan$^R$* and *CM$^R$),* and P22 bacteriophage was transduced into the SG3005 strain carrying the genome containing *ssrAB::kan$^R$,* thereby producing a bacteriophage containing *ssrAB::kan$^R$* gene. The inactivation of the antibiotic-resistant genes was completed through the introduction of pCP20 (helper plasmid) transformation method inducing the expression of FLP recombinant enzyme, and the methods for deleting antibiotic-resistant genes in all the mutant *Salmonella gallinarum* constructed for the present invention are the same as each other.

The Δ*relA*, Δ*spoT, ssrAB: :kan$^R$* mutant (SG4041) was constructed by the transduction of a bacteriophage carrying *ssrAB::kan$^R$* gene into the inactivated ppGpp-deleted mutant. As for the presence or absence of the bacteriophage (Gifsy *2 prophage)* genome in the wild-type *Salmonella gallinarum* (clinical isolate), the presence of one type of bacteriophage genome (Gifsy *2 prophage)* was confirmed by the whole genome sequencing method of the wild-type *Salmonella gallinarum.* For attenuation, the corresponding prophage genome was deleted by replacing the prophage gene of the genome of SG4021 with chloramphenicol (cm)-resistant gene (Gifsy *2 prophage::cm$^R$)* through the Lambda-Red homologous recombination method, and Gifsy 2 *prophage* genome-deleted mutant *Salmonella gallinarum* was constructed (SG4042).

For the construction of the ppGpp-, *ssrAB-, and* Gifsy 2 prophage-deleted mutant *Salmonella gallinarum,* a bacteriophage carrying Gifsy *2 prophage::cm$^R$* (utilizing P22 and SG4042 strain) was first prepared, and then transduced into the SG4041 strain (Δ*relA,* Δ*spoT, ssrAB: :kan$^R$,* Gifsy *2 prophage::cm$^R$;* SG4043). To avoid the problems of excessive antibiotic resistance in the development of anticancer agents of attenuated *Salmonella gallinarum,* SG4044 (Δ*relA,* Δ*spoT,* Δ*ssrAB,* ΔGifsy *2 prophage*) strain in which all the antibiotic-resistant genes were removed in the SG4043 strain was developed, and for evaluation of efficacy and values of the attenuated Salmonella strains constructed in the present invention, strains containing only the chloramphenicol-resistant gene were constructed (Δ*relA,* Δ*spoT,* Δ*ssrAB,* Gifsy 2 *prophage::cm$^R$;* SG4046) .

To develop the SG4046 strain, the SG4045 (Δ*relA,* Δ*spoT,* Δ*ssrAB*) in which a kanamycin-resistant gene was deleted was constructed and utilized. In the *glmS* open reading frame, the gene carrying a kanamycin-resistant gene *(kan$^R$)* was produced by polymerase chain reaction (PCR) using a pair of 60-nt primers, each including a 40-nt homology extension and a 20-nt priming sequence, with pKD13 serving as a template. The nucleotide sequences and sequence numbers of the primers are shown in Table 2 below. The bacterial chromosomal DNA of wild-type *Salmonella gallinarum* was used as a template used for PCR.

TABLE 2

| Primer | | Primer nucleotide sequence (5'→ 3') | SEQ ID NO |
|---|---|---|---|
| *relA::k anR* | Forward | GTGGATCGCAAGCCTGGGAATTTCCAGCCAGCA GTCGTGTGAGCGCTTAGGTGTAGGCTGGAGCTG CTTC | SEQ ID NO: 1 |
| | Reverse | GTGCAGTCGCCGTGCATCAATCACATCCGGCAC CTGGTTCAGCTTACCGAATTCCGGGGATCCGTC GACC | SEQ ID NO: 2 |
| *spot::c m$^R$* | Forward | TTAAGCGTCTTCGGCAGGCGTATCTCGTTGCAC GTGACGCTCACGAGGGCTGTAGGCTGGAGCTGC TTC | SEQ ID NO: 3 |
| | Reverse | GCCAGATGTACGCGATCGCGTGCGGTAAGGCGA ATAAAGGTACTATAGACCATATGAATATCCTCC TTAG | SEQ ID NO: 4 |
| *ssrAB:: kan$^R$* | Forward | ATGAATTTGCTCAATCTCAAGAATACGCTGCAA ACATCTTGTGTAGGCTGGAGCTGCTTC | SEQ ID NO: 5 |
| | Reverse | TTAATACTCTATTAACCTCATTCTTCGGGCACA GTTAAGTATTCCGGGGATCCGTCGACC | SEQ ID NO: 6 |
| *Δ* Gifsy *2 prophag e:: cm$^R$* | Forward | TATAAATTTAATATACTAACCAGTAACCATATC AGTTATGACAGACAGGCGTGTAGGCTGGAGCTG CTTC | SEQ ID NO: 7 |
| | Reverse | AGAACACAGAGAAAATGTCATTGCATATGGTCA AAAAATAGACATATTTAGGTCCATATGAATATC CTCCTTAGTTCCTATTCC | SEQ ID NO: 8 |

The purified PCR product was transformed into attenuated *Salmonella* (Δ*relA,* Δ*spoT;* SG4023) containing pKD46 by electroporation, and this was used to construct a *glmS* gene-deleted attenuated *Salmonella gallinarum* (Δ*relA,* Δ*spoT, glmS*::kan$^R$; SG4030). For the construction of the *glmS* gene-deleted attenuated *Salmonella gallinarum* (Δ*relA,* Δ*spoT,* Δ*ssrAB,* ΔGifsy *2 prophage, glmS::kan$^R$*), P22 phage containing *glmS::kan$^R$* was first prepared (using the strain SG4030), and then the prepared P22 phage containing *glmS::kan$^R$* was transduced into SG4044 (Δ*relA,* Δ*spoT,* Δ*ssrAB,* ΔGifsy *2 prophage*) to construct the Δ*relA,* Δ*spoT, ΔssrAB,* ΔGifsy *2 prophage, glmS::kan$^R$* (SG4047) mutant *Salmonella gallinarum* strain.

To avoid the problems of excessive antibiotic resistance in the development of anticancer drugs of attenuated *Salmonella gallinarum,* a kanamycin-resistant gene of SG4047 was deleted by the same method as above, thereby

constructing the ΔrelA, ΔspoT, ΔssrAB, ΔGifsy *2 prophage, ΔglmS Salmonella gallinarum* strain (SG4048) as a final strain.

Since Salmonella strains particularly tend to release plasmids containing and carrying reporter genes that are not required for survival in animals, the utilization of antibiotic-resistant genes is not possible for selective determinants. Therefore, a *glmS*-deleted mutant phenotype soluble in the animal system was used due to the lack of D-glucosamine (GlcN) or N-acetyl-D-glucosamine (GlcNAc), which is a peptidoglycan synthesis component, as an essential nutrient for proliferation.

**1-3. *GlmS*⁺pLux plasmid**

To evaluate tumor targeting ability through bioluminescence and complement the *glmS* gene deletion on the chromosome, a balanced lethal host vector system incorporating *glmS* gene of the *Salmonella gallinarum* strain was constructed.

Specifically, pLux containing the lux operon (luxCDABE, approximately 9.5 kbp) of *Photobacterium leiognathi* was inserted into the *XbaI* restriction enzyme site in the pUC19 plasmid backbone. To construct a plasmid containing both Lux operon cassette and *glmS,* the *glmS* gene of the *Salmonella gallinarum* strain was amplified by specific primers. The nucleotide sequences and sequence numbers of the primers are shown in Table 3 below. The bacterial chromosomal DNA of wild-type *Salmonella gallinarum (SG4021)* was used as a template used for PCR.

TABLE 3

| Primer | | Primer nucleotide sequence (5'→3') | SEQ ID NO |
|---|---|---|---|
| *glmS* | Forward | ACGCGGTCGACATGTGTGGAATTGTTGGCGCT | SEQ ID NO: 9 |
| | Reverse | ACGCGGTCGACTTACTCTACGGTAACCGATTTCGCC | SEQ ID NO: 10 |

Both ends of the pLux vector and the amplified 1.8 kbp fragment were digested with *Sal I* restriction enzyme and ligated by T4 DNA ligase, thereby preparing *GlmS⁺pLux.* The *glmS*-deleted mutant *Salmonella gallinarum* strain transformed with the prepared *GlmS*⁺pLux can survive even in an environment lacking D-glucosamine (GlcN) or N-acetyl-D-glucosamine (GlcNAc) and exhibit bioluminescence, so that the strain can be analyzed by optical bioluminescence images.

**1-4. Tumor cell lines**

Murine-derived CT26 (colon tumor cell line), 4T-1 (breast tumor cell line), B16F10 (melanoma cell line), and LL2 (LLC-1, lung tumor cell line) were purchased from the American type culture collection, and murine-derived Panc02 (pancreatic tumor cell line) was purchased from National cancer institute _Division of cancer treatment & Diagnosis (DCTD). The CT26, 4T-1, LLC-1, and Panc02 cell lines were grown in high-glucose DMEM (Dulbecco's Modified Eagle's Medium) containing 10% fetal bovine serum (FBS) and 1% penicillin-streptomycin, and the B16F10 cell line was grown in RPMI1640 containing 10% fetal bovine serum and 1% penicillin-streptomycin.

**1-5. Plasmid stability measurement**

The cells were subcultured (1/1000) overnight in flesh LB medium that was replenished every 12 hours. Samples were taken and diluted every 24 hours. A suitable volume was spread three times over GlcNAc-supplemented LB plates, regardless of the presence or absence of ampicillin. The total viable cells (colony forming units, CFU) and the fraction of Salmonella strain cells carrying the plasmid were calculated by using the number of colonies.

**1-6. Construction of Mouse models**

The various tumor cell lines that were cultured were hetero-transplanted into the right thigh of each mouse to construct mouse tumor models. Female mice aged 5 to 8 weeks weighing 20 to 30 kg were purchased from Samtako company (Korea). All the animals were managed, experimented, and euthanized according to approved protocols.

Mice with subcutaneous tumor were constructed as follows. The tumor cell lines of Example 1-4 cultured in vitro were harvested and suspended in 30 ml of PBS, followed by subcutaneous injection of $1\times10^6$ cells of each of the CT26, 4T-1, LLC-1, and B16F10 cell lines and $1\times10^7$ cells of the Panc02 cell line into the right thigh of each mouse. The CT26 and 4T-1 cell lines were transplanted into Balb/C mice, and the LLC-1, Panc02, and B16F10 cell lines were transplanted into C57/BL6 mice.

When the tumor size reached 60 $mm^3$ after the transplantation of tumor cells, the glmS-deleted mutant attenuated *Salmonella gallinarum* strain (SG4050) transformed with the *GlmS*$^+$pLux of Example 1-3 was intravenously injected at about $5\times10^8$ CFU, and the ppGpp-, *ssrAB-,* and Gifsy 2 prophage-deleted *Salmonella gallinarum* strain (SG4044 or SG4046, $5\times10^8$ CFU) or ppGpp-deleted *Salmonella typhimurium* strain (SMR2130, $1\times10^7$ CFU) of Example 1-2 was administered via tail vein.

**1-7. Evaluation of distribution of Salmonella strains in mouse internal organs**

To evaluate the numbers of live cells of the Salmonella stains, ΔppGpp *Salmonella typhimurium* (SMR2130) at about $1\times10^7$ CFU and ΔppGpp, *ΔssrAB,* Gifsy 2 *prophage Salmonella gallinarum* strain (SG4046) at $5\times10^8$ CFU were intravenously administered into mouse groups (n = 5). Days 1, 3, 5, 10, and 15 after the intravenous administration, the mice were sacrificed to collect organs, and organ tissues were homogenized in sterile PBS containing 0.05% Tween-20 by using a homogenizer. The *Salmonella* strains were recovered from the homogenates and plated onto agar plates containing 50 pg/ml kanamycin and 15 ug/ml chloramphenicol to be quantified.

**1-8. Tumor sizes and mouse survival rates**

Tumor volumes were calculated according to the following equation.

[Equation]

$$\text{Tumor volume } (mm^3) = (\text{tumor length} \times \text{tumor height} \times \text{tumor width})/2$$

All animal experiments were approved by the Chonnam National University Institutional Animal Care and Use Committee (NO. CNU IACUC-H-2016-15), and according to the instruction, the mice with a tumor volume of 1,500 $mm^3$ or more were sacrificed, and the survival rates of mice were evaluated according to the Gehan-Breslow-Wilcoxon test.

**1-9. Optical bioluminescent imaging**

Imaging was performed using the bioluminescence of the *Salmonella* strain as an index for tumor targeting. As for tumor targeting ability, the mice were anesthetized with 2% isoflurane and then placed in a light-blocked chamber of IVIS100 (Caliper, Hopkinton, MA, USA) equipped with a cooled charged couple detector (CCD) camera. The photons released from luciferase-expressing *Salmonella* strains were collected and integrated for 1 minute. Pseudo color images representing the photon count were overlaid on images of mice by using Living image software version 2.25 (Xenogen-Caliper, Hopkinto, MA).

**1-10. Statistical Analysis**

Statistical analysis was performed using SPSS 18.0 statistical package (SPSS Inc., Chicago, IL, USA). Statistical significance of tumor growth between a control group and a test group was determined using Two-Tail Student's t-test. P value < 0.05 was considered statistically significant, and all data are expressed as mean ± SD.

**[Example 2]**

**In vivo toxicity of attenuated Salmonella *gallinarum* strains and plasmid stability therefor**

**2-1. Amino acid requirements of *Salmonella gallinarum* strains**

As shown in FIG. 1, the wild-type *Salmonella gallinarum* was grown only in the minimal medium supplemented with leucine (Leu), and the growth thereof was improved by addition of arginine (Arg) and phenylalanine (Phe). The ppGpp-deleted attenuated *Salmonella gallinarum* was grown in the minimal medium supplemented with isoleucine (Ile), lysine (Lys), serine (Ser), and valine (Val). Therefore, the ppGpp-deleted *Salmonella gallinarum* was confirmed to have less amino acid requirements compared with the ppGpp-deleted *Salmonella typhimurium* (LT2) (Tedin and Norel, J Bacteriol, 2001, 183:6184-6196).

### 2-2. In vivo toxicity of *Salmonella gallinarum* strain

To apply *Salmonella gallinarum* strains to bacterial cancer therapy, wild-type *Salmonella gallinarum* strains was first injected through intra vein (IV), and then mouse survival rates were analyzed.
As shown in FIG. 2 below, even though causing typhoid only in fowls, the intravenous injection of the wild-type *Salmonella gallinarum* strain (SG4021) resulted in death of all mice at a dose of about $10^5$ CFU or more.

### 2-3. Dosage according to in vivo toxicity of attenuated Salmonella gallinarum strain

Since the wild-type *Salmonella gallinarum* strains were confirmed to carry in vivo mouse toxicity in Example 2-2, the *Salmonella gallinarum* strain was attenuated by deletion of ppGpp, *ssrAB,* and Gifsy *2 prophage.*
Specifically, the ΔppGpp, *ΔssrAB,* Gifsy *2 prophage Salmonella gallinarum* strain (SG4046) and the ΔppGpp *Salmonella typhimurium* strain (SMR2130) were intravenously administered into Blab/C mice to analyze mouse survival rates.
In examining the strain distribution for each organ, SG4046 containing an antibiotic marker was used to check accurate cell counts.
As shown in FIGS. 3 and 4, the mice were observed to survive up to $5\times10^8$ CFU of the ΔppGpp, *ΔssrAB,* Gifsy *2 prophage Salmonella gallinarum* strain (SG4046), and the mice were observed to survive up to about $1\times10^7$ CFU of the ΔppGpp *Salmonella typhimurium* strain (SMR2130).
From the experimental results of attenuated *Salmonella typhimurium* (A1-R) with confirmed tumor targeting ability and anti-tumor effect, the modified lipopolysaccharide, msbB-deleted mutant (VNP20009) and ΔrfaG/ΔrfaD double mutant strains had a treatment range at about $1\times10^6$ to $1\times10^7$ CFU. Therefore, the attenuated *Salmonella typhimurium* had about 15 times higher in vivo stability than the attenuated *Salmonella typhimurium,* indicating more excellent stability.
Considering the effective dosage of *Escherichia coli,* which was recognized as having secured in vivo stability, was about $1\times10^8$ CFU (route of administration: intra vein, IV), the in vivo stability of the attenuated *Salmonella gallinarum* of the present invention was remarkably excellent.

### 2-4. Plasmid stability

To examine the stability of transformed plasmids, the same procedure as in Example 1-5 was carried out to evaluate whether the plasmids were lost.
As shown in FIG. 5, 99% of the plasmids carried by the wild-type *Salmonella gallinarum* strain (SG4021) were lost by Day 4 while the plasmids carried by the *glmS*-deleted *Salmonella gallinarum* strain (SG4050) were completely maintained.
To visualize *Salmonella gallinarum* strains in tumorformed mice in subsequent experiments, the *Salmonella gallinarum* strain (SG4050) transformed with the *GlmS*$^+$pLux of Example 1-3 and having a mutant *glmS* gene on the genome thereof was used.

## [Example 3]

### Tumor targeting of attenuated *Salmonella gallinarum* strain

To examine the tumor targeting of the ΔppGpp, *ΔssrAB,* ΔGifsy *2 prophage Salmonella gallinarum* strain, bioluminescent signals of the attenuated *Salmonella gallinarum* strain were imaged according to Example 1-9.
As shown in FIG. 6, the in vivo luminescent signals were detected mainly in the endothelial organs (liver and spleen) immediately after the injection of the ΔppGpp, Δ*ssrAB,* ΔGifsy *2 prophage,* Δ*glmS Salmonella gallinarum* strain (SG4050) (20 min, 0 day post injection (dpi)), but the in vivo bioluminescent signals at 3 days post injection (3 dpi) were detected only in the transplanted tumor tissues. Especially, the attenuated *Salmonella gallinarum* strain was observed to target all the tumors transplanted in four different mouse lines.
Therefore, the ΔppGpp, *ΔssrAB,* ΔGifsy *2 prophage,* Δ*glmS Salmonella gallinarum* strain (SG4048) showed an excellent tumor targeting effect.

**[Example 4]**

## Anticancer effect of ΔppGpp, *ΔssrAB,* ΔGifsy 2 *prophage Salmonella* gallinarum strain in CT26 tumor mouse model

### 4-1. Anti-tumor effect of attenuated Salmonella *gallinarum* and extending of survival period thereof

CT26 cell-transplanted Blab/C mice were intravenously administered with the ΔppGpp, *ΔssrAB,* ΔGifsy *2 prophage Salmonella gallinarum* strain (SG4044) at about $5\times10^8$ CFU while a PBS treatment group and the ΔppGpp *Salmonella typhimurium* strain ($1\times10^7$ CFU, SMR2130) were used as control groups. The in vivo anti-tumor activity of the ΔppGpp, Δ*ssrAB,* ΔGifsy *2 prophage Salmonella gallinarum* strain was analyzed according to Examples 1-8 and 1-10.

As shown in FIG. 7, as a result of treating a colorectal tumor-forming mice with the ΔppGpp, *ΔssrAB,* ΔGifsy *2 prophage Salmonella gallinarum* strain (SG4044) at about $5\times10^8$ CFU, the tumor growth was significantly delayed compared with those in the control groups.

As shown in FIG. 8, the survival period in the group treated with the ΔppGpp, *ΔssrAB,* ΔGifsy *2 prophage Salmonella gallinarum* strain (SG4044) was approximately at least twice as long as those in the control groups. The measured extended survival period was about 24 days in the ΔppGpp *Salmonella typhimurium* strain treatment group and 15 days in the PBS treatment group. The mean survival period in the group treated with the ΔppGpp, *ΔssrAB,* ΔGifsy *2 prophage Salmonella gallinarum* strain was 38 days.

### 4-2. Distribution pattern of *Salmonella gallinarum* strain in mice

To examine the distribution pattern of the *Salmonella gallinarum* strain in mice, the Salmonella cell count distributions over time in mouse organs were evaluated according to Example 1-7.

As shown in FIG. 9, in both groups administered with the ΔppGpp, *ΔssrAB,* Gifsy *2 prophage Salmonella gallinarum* strain ($5\times10^8$, SG4046) and the ΔppGpp *Salmonella typhimurium* strain ($1\times10^7$, SMR2130), the largest number of *Salmonella* strain cells was observed within the tumor at 3 days post injection (3 dpi), and the number of bacterial cells was maintained at similar levels for 10 days and gradually decreased after 10 days within the tumor.

At 1 day post injection (1 dpi), the attenuated *Salmonella typhimurium* (SMR2130) was present at $10^6$ CFU/g in the liver, $10^7$ CFU/g in the spleen, $10^4$ CFU/g in the lung, kidney, and heart, and $10^2$ CFU/g in the blood. These cell counts in the normal tissues were detected at the same level in the other tissues excluding a blood sample during the experimental period. On the other hand, the cell count of the attenuated *Salmonella gallinarum* (SG4046) was detected at a similar level to the attenuated *Salmonella typhimurium* (SMR2130) at 1 day post injection (1 dpi), but thereafter gradually decreased to be completely eliminated on Day 15.

Accordingly, the ΔppGpp, *ΔssrAB,* ΔGifsy *2 prophage Salmonella gallinarum* strain of the present invention exhibits an anti-tumor effect by specifically targeting tumor, and thus can be used for anti-tumor application without the fear of side effects in other organs.

BUDAPEST TREATY ON THE INTERNATIONAL RECOGNITION OF THE DEPOSIT OF MICROORGANISMS FOR THE PURPOSE OF PATENT PROCEDURE

INTERNATIONAL FORM

# RECEIPT IN THE CASE OF AN ORIGINAL DEPOSIT

issued pursuant to Rule 7.1

TO: **Industry Foundation of Chonnam National University**

Industry Foundation of Chonnam National University
77, Yongbong-ro, Buk-gu, Gwangju
Republic of Korea

| I. IDENTIFICATION OF THE MICROORGANISM | |
|---|---|
| Identification reference given by the DEPOSITOR:<br><br>***Salmonella. gallinarum*** **(SG4021)** | Accession number given by the INTERNATIONAL DEPOSITARY AUTHORITY:<br><br>**KCTC 13985BP** |

| II. SCIENTIFIC DESCRIPTION AND/OR PROPOSED TAXONOMIC DESIGNATION |
|---|
| The microorganism identified under I above was accompanied by:<br>[ ] a scientific description<br>[ ] a proposed taxonomic designation<br>(Mark with a cross where applicable) |

| III. RECEIPT AND ACCEPTANCE |
|---|
| This International Depositary Authority accepts the microorganism identified under I above, which was received by it on **October 8, 2019.** |

| IV. RECEIPT OF REQUEST FOR CONVERSION |
|---|
| The microorganism identified under I above was received by this International Depositary Authority on and a request to convert the original deposit to a deposit under the Budapest Treaty was received by it on |

| V. INTERNATIONAL DEPOSITARY AUTHORITY | |
|---|---|
| Name: **Korean Collection for Type Cultures**<br><br>Address: Korea Research Institute of Bioscience and Biotechnology (KRIBB)<br>181, Ipsin-gil, Jeongeup-si, Jeolllabuk-do 56212<br>Republic of Korea | Signature(s) of person(s) having the power to represent the International Depositary Authority or of authorized official(s):<br><br>Song-Gun Kim<br><br>KIM, Song-Gun, Director<br>Date: **October 29, 2019** |

Form BP/4 (KCTC Form 17) sole page

(Reissue)

BUDAPEST TREATY ON THE INTERNATIONAL RECOGNITION OF THE DEPOSIT OF MICROORGANISMS FOR THE PURPOSE OF PATENT PROCEDURE

INTERNATIONAL FORM

# RECEIPT IN THE CASE OF AN ORIGINAL DEPOSIT

issued pursuant to Rule 7.1

TO: **Industry Foundation of Chonnam National University**
Industry Foundation of Chonnam National University
77, Yongbong-ro, Buk-gu, Gwangju
Republic of Korea

| I. IDENTIFICATION OF THE MICROORGANISM | |
|---|---|
| Identification reference given by the DEPOSITOR:<br><br>***S. gallinarum ΔrelA, ΔspoT, ΔssrAB, ΔGifsy 2 prophage, ΔglmS* (SG4048)** | Accession number given by the INTERNATIONAL DEPOSITARY AUTHORITY:<br><br>**KCTC 14542BP** |

II. SCIENTIFIC DESCRIPTION AND/OR PROPOSED TAXONOMIC DESIGNATION

The microorganism identified under I above was accompanied by:

[ ] a scientific description

[ ] a proposed taxonomic designation

(Mark with a cross where applicable)

III. RECEIPT AND ACCEPTANCE

This International Depositary Authority accepts the microorganism identified under I above, which was received by it on **April 20, 2021.**

IV. RECEIPT OF REQUEST FOR CONVERSION

The microorganism identified under I above was received by this International Depositary Authority on and a request to convert the original deposit to a deposit under the Budapest Treaty was received by it on

| V. INTERNATIONAL DEPOSITARY AUTHORITY | |
|---|---|
| Name: **Korean Collection for Type Cultures**<br><br>Address: Korea Research Institute of Bioscience and Biotechnology (KRIBB)<br>181, Ipsin-gil, Jeongeup-si, Jeolllabuk-do 56212<br>Republic of Korea | Signature(s) of person(s) having the power to represent the International Depositary Authority or of authorized official(s):<br><br>Song-Gun Kim<br><br>KIM, Song-Gun, Director<br>Date: **April 27, 2021** |

Form BP/4 (KCTC Form 17) sole page

(Reissue)

BUDAPEST TREATY ON THE INTERNATIONAL RECOGNITION OF THE DEPOSIT OF MICROORGANISMS FOR THE PURPOSE OF PATENT PROCEDURE

INTERNATIONAL FORM

# RECEIPT IN THE CASE OF AN ORIGINAL DEPOSIT

issued pursuant to Rule 7.1

TO: **Industry Foundation of Chonnam National University**

Industry Foundation of Chonnam National University

77, Yongbong-ro, Buk-gu, Gwangju

Republic of Korea

| I. IDENTIFICATION OF THE MICROORGANISM | |
|---|---|
| Identification reference given by the DEPOSITOR:<br><br>***S. gallinarum Δ relA,Δ spot, Δ ssrAB, Δ Gifsy 2 prophage*** **(SG4044)** | Accession number given by the INTERNATIONAL DEPOSITARY AUTHORITY:<br><br>**KCTC 14541BP** |

II. SCIENTIFIC DESCRIPTION AND/OR PROPOSED TAXONOMIC DESIGNATION

The microorganism identified under I above was accompanied by:

[ ] a scientific description

[ ] a proposed taxonomic designation

(Mark with a cross where applicable)

III. RECEIPT AND ACCEPTANCE

This International Depositary Authority accepts the microorganism identified under I above, which was received by it on **April 20, 2021.**

IV. RECEIPT OF REQUEST FOR CONVERSION

The microorganism identified under I above was received by this International Depositary Authority on and a request to convert the original deposit to a deposit under the Budapest Treaty was received by it on

| V. INTERNATIONAL DEPOSITARY AUTHORITY | |
|---|---|
| Name: **Korean Collection for Type Cultures**<br><br>Address: Korea Research Institute of Bioscience and Biotechnology (KRIBB)<br>181, Ipsin-gil, Jeongeup-si, Jeolllabuk-do 56212<br>Republic of Korea | Signature(s) of person(s) having the power to represent the International Depositary Authority or of authorized official(s):<br><br>Song-Gun Kim<br><br>KIM, Song-Gun, Director<br>Date: **April 20, 2021** |

Form BP/4 (KCTC Form 17) sole page

## Claims

1. An attenuated *Salmonella gallinarum* strain in which a gene encoding guanosine tetraphosphate (ppGpp) synthetase, a gene inducing the function of a type III secretion system (T3SS), and a Gifsy *2 prophage* gene are deleted.

2. The attenuated *Salmonella gallinarum* strain of claim 1, wherein *glmS* gene is deleted.

3. The attenuated *Salmonella gallinarum* strain of claim 1, wherein the gene encoding guanosine tetraphosphate (ppGpp) synthetase is *relA* or *spoT.*

4. The attenuated *Salmonella gallinarum* strain of claim 1, wherein the gene inducing the function of a type III secretion system (T3SS) is *ssrA, ssrB,* or *ssrAB.*

5. The attenuated *Salmonella gallinarum* strain of claim 1, wherein the strain has deletions of *relA, spoT, ssrAB,* and Gifsy *2 prophage* genes in the *Salmonella gallinarum* SG4021 strain (accession number: KCTC13985BP).

6. The attenuated *Salmonella gallinarum* strain of claim 1, wherein the strain has deletions of *relA, spoT, ssrAB,* Gifsy *2 prophage, and glmS* genes in the *Salmonella gallinarum* SG4021 strain (accession number: KCTC13985BP).

7. The attenuated *Salmonella gallinarum* strain of claim 1, wherein the strain is the *Salmonella gallinarum* SG4044 strain (accession number: KCTC14541BP).

8. The attenuated *Salmonella gallinarum* strain of claim 1, wherein the strain is the *Salmonella gallinarum* SG4048 strain (accession number: KCTC14542BP).

9. The attenuated *Salmonella gallinarum* strain of claim 2, wherein the strain is transformed with a plasmid carrying *glmS* gene operatively linked to a promoter.

10. The attenuated *Salmonella gallinarum* strain of claim 1, wherein the strain further comprises a luminescent gene.

11. The attenuated *Salmonella gallinarum* strain of claim 9, wherein the luminescent gene is *luxCDABE.*

12. A pharmaceutical composition for diagnosis or treatment of a tumor, the composition comprising the strain of any one of claims 1 to 11.

13. The pharmaceutical composition of claim 12, wherein the tumor is a solid tumor.

14. The pharmaceutical composition of claim 12, wherein the tumor is an adenocarcinoma.

15. The pharmaceutical composition of claim 12, wherein the tumor is any one cancer selected from the group consisting of colorectal cancer, pancreatic cancer, lung cancer, skin cancer, and breast cancer.

16. A method for providing information for tumor diagnosis, the method comprising:

    administering the strain of claim 10 or 11 to a subject;
    detecting the bioluminescence of the strain; and
    determining that a tumor is present in the subject when the bioluminescence is detected.

【Figure 1】

**Amino acid requirements of Δ*relA* Δ*spoT* mutants**

| Strain | Ala | Arg | Asn | Asp | Cys | Gln | Glu | Gly | His | Ile | Leu | Lys | Met | Phe | Pro | Ser | Thr | Trp | Tyr | Val |
|---|---|---|---|---|---|---|---|---|---|---|---|---|---|---|---|---|---|---|---|---|
| **S.Gallinarum** | | | | | | | | | | | | | | | | | | | | |
| Wild type (SG4021) | + | ± | + | + | + | + | + | + | + | + | - | + | + | ± | + | + | + | + | + | + |
| Δ*relA* Δ*spoT* (SG4023) | + | ± | + | + | + | + | + | + | + | - | - | - | + | ± | + | - | + | + | + | - |
| Δ*relA*Δ*spoT* Δ*ssrA* *B* Δ*prophage* (SG4044) | + | ± | + | + | + | + | + | + | + | - | - | - | + | ± | + | - | + | + | + | - |
| Δ*relA*Δ*spoT* Δ*ssrA* *B* (SG4045) | + | ± | + | + | + | + | + | + | + | - | - | - | + | ± | + | - | + | + | + | - |

Amino acid requirements were scored after downshift of an overnight LB agar plate to M9 minimal glucose plates containing combinations of 19 amino acids at 100 μg/ml with 1 of the full set of 20 o mitted. Control plates included one with the full complement of 20 amino acids and one without am ino acid supplements (M9 minimal glucose).

+, normal growth
±, poor growth or microcolonies
-, no visible growth after 24 to 48 h of incubation.

【Figure 2】

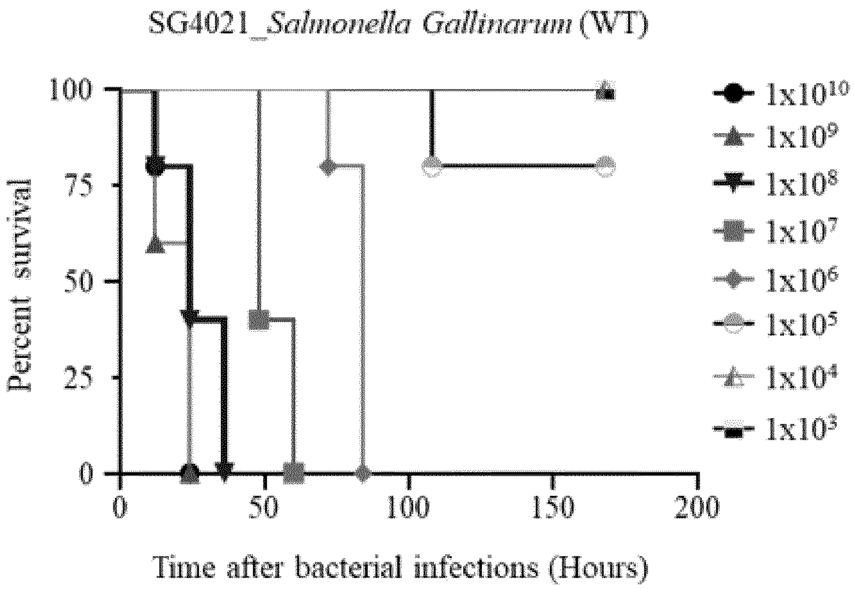
SG4021_Salmonella Gallinarum (WT)
Percent survival
100
75
50
25
0
0
50
100
150
200
Time after bacterial infections (Hours)
1x10^10
1x10^9
1x10^8
1x10^7
1x10^6
1x10^5
1x10^4
1x10^3

【Figure 3】

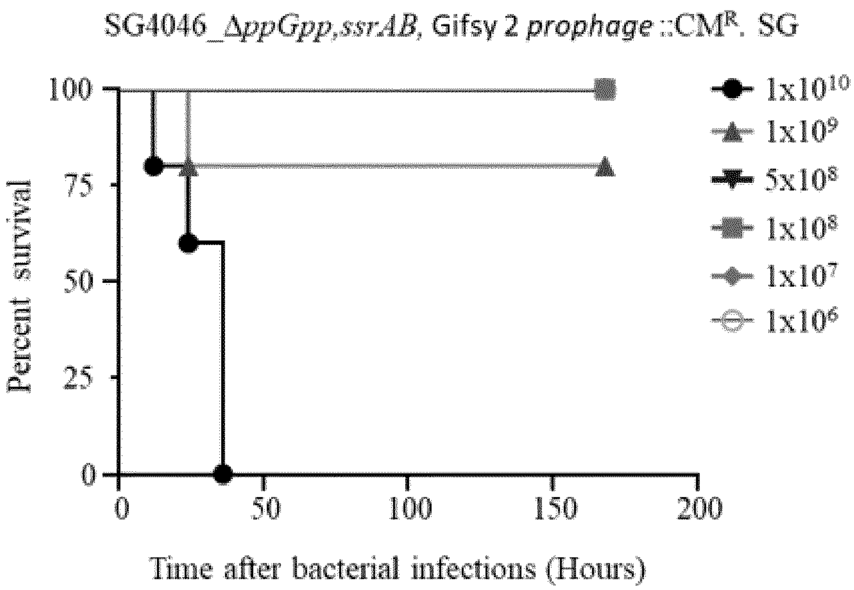
SG4046_ΔppGpp,ssrAB, Gifsy 2 prophage ::CMR. SG
Percent survival
100
75
50
25
0
0
50
100
150
200
Time after bacterial infections (Hours)
1x10^10
1x10^9
5x10^8
1x10^8
1x10^7
1x10^6

【Figure 4】

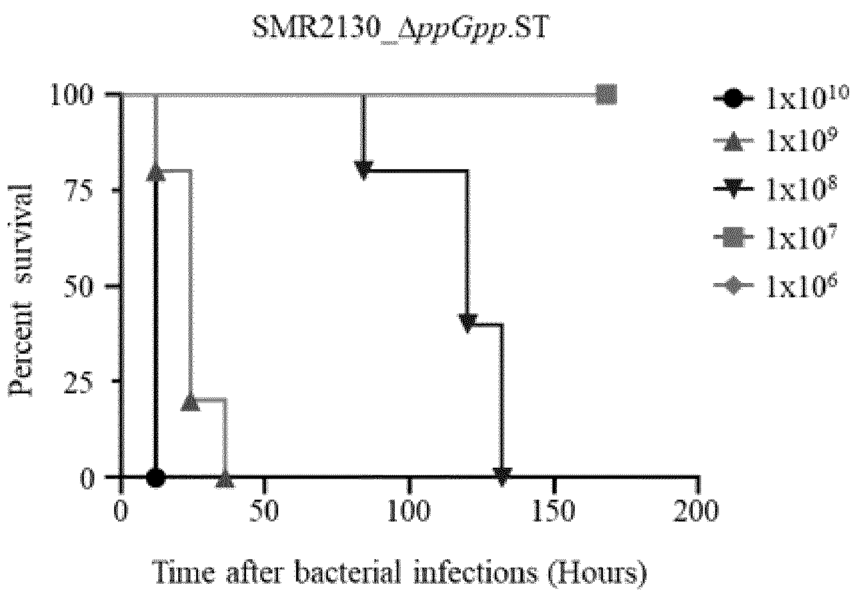
SMR2130_ΔppGpp.ST
Percent survival
100
75
50
25
0
0
50
100
150
200
Time after bacterial infections (Hours)
1x10¹⁰
1x10⁹
1x10⁸
1x10⁷
1x10⁶

【Figure 5】

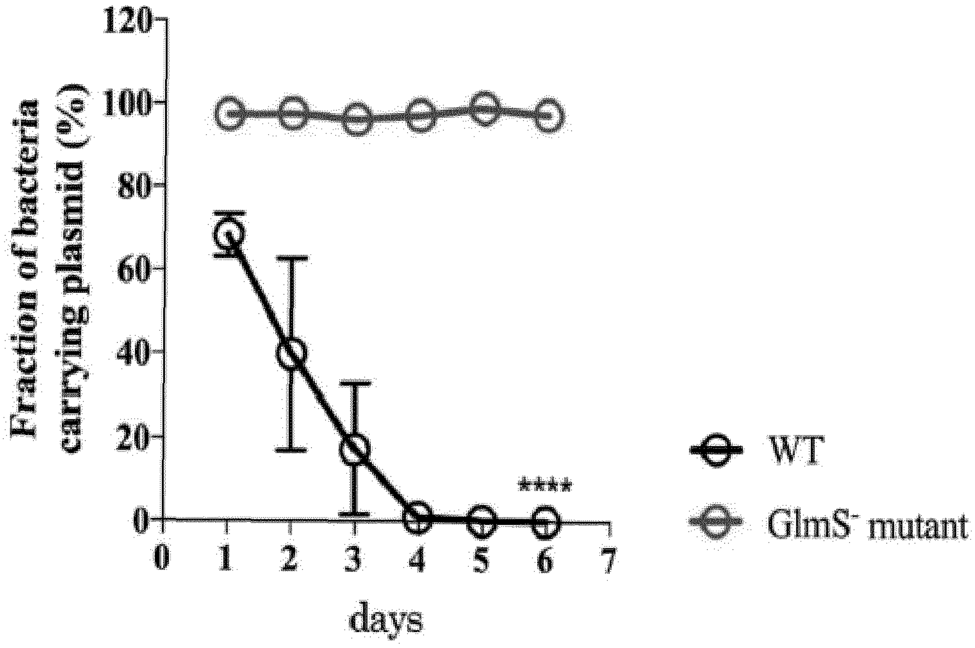
120
100
80
60
40
20
0
Fraction of bacteria carrying plasmid (%)
0
1
2
3
4
5
6
7
days
****
WT
GlmS- mutant

【Figure 6】

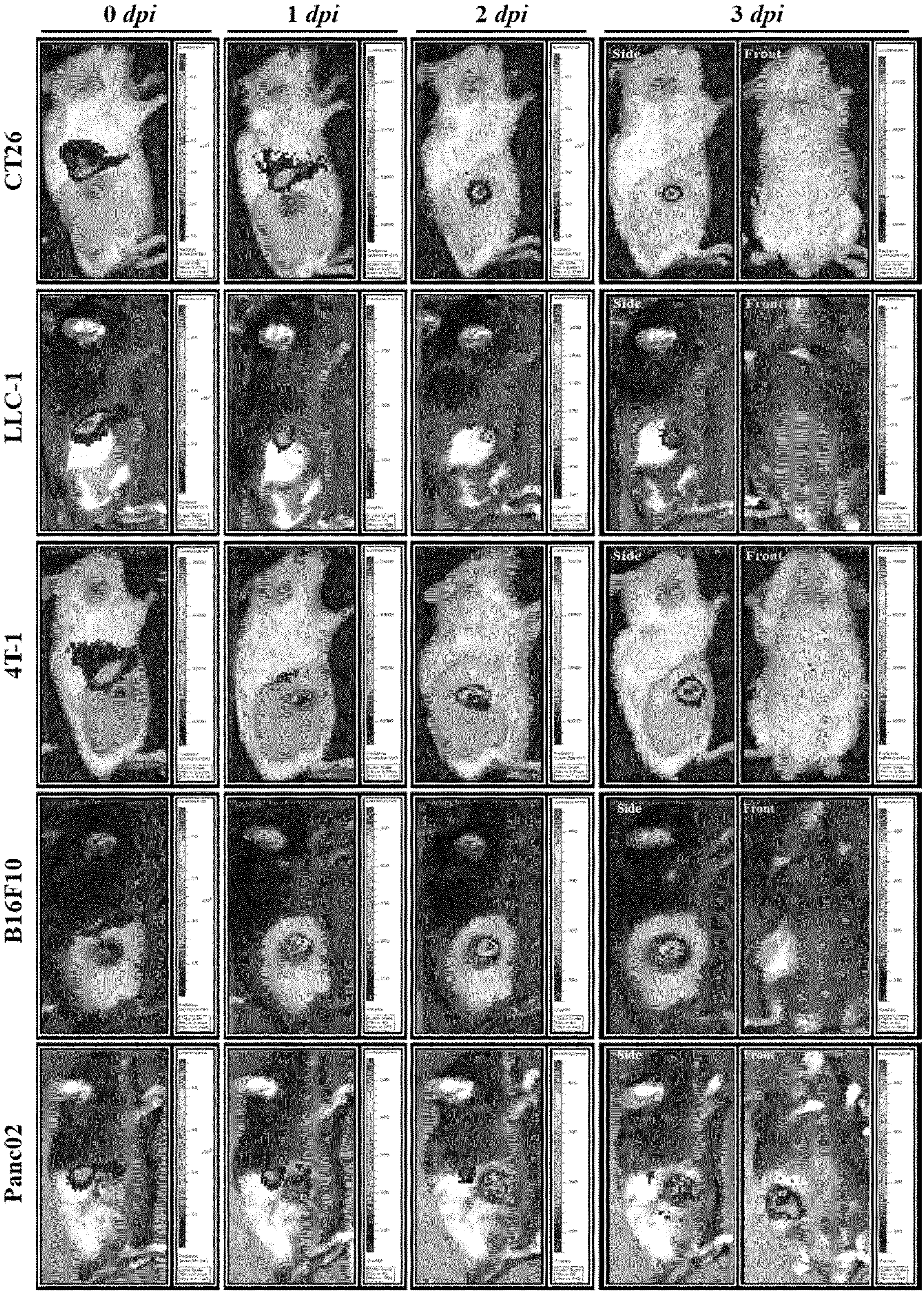

【Figure 7】

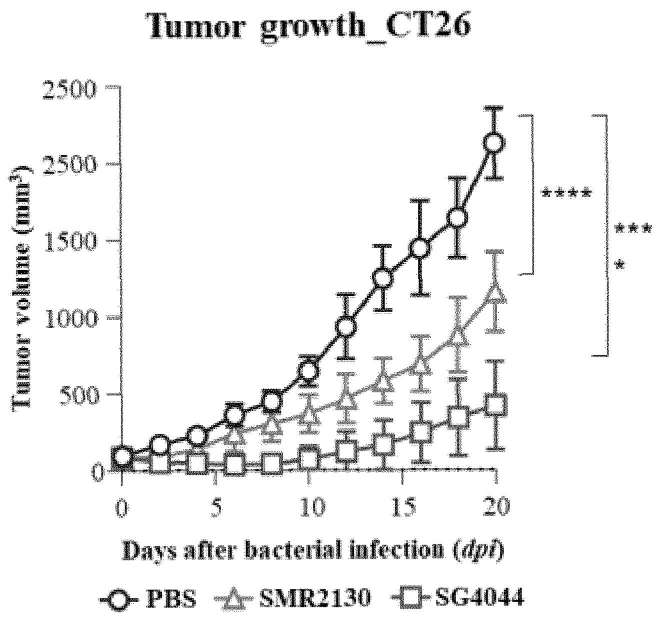
Tumor growth_CT26
2500
2500
1500
1000
500
0
Tumor volume (mm³)
0
5
10
15
20
Days after bacterial infection (dpi)
****
***
*
PBS
SMR2130
SG4044

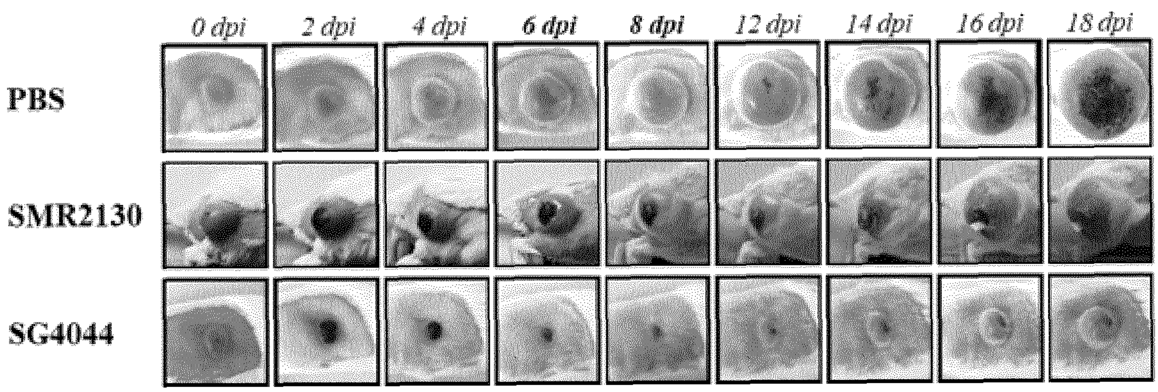
0 dpi
2 dpi
4 dpi
6 dpi
8 dpi
12 dpi
14 dpi
16 dpi
18 dpi
PBS
SMR2130
SG4044

【Figure 8】

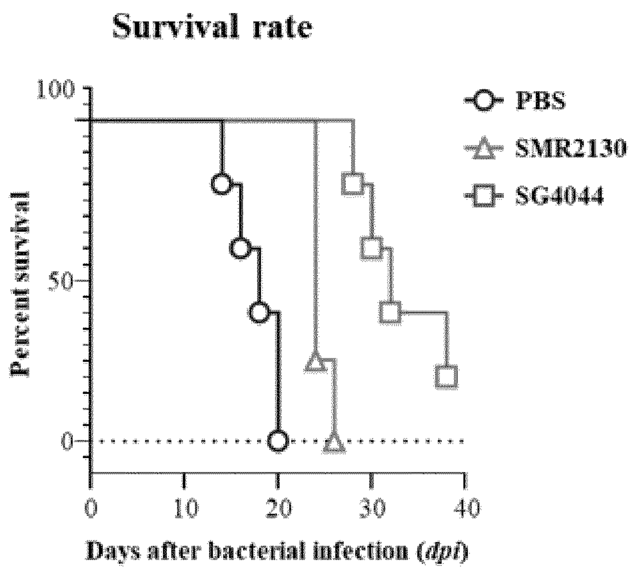
Survival rate
100
50
0
Percent survival
PBS
SMR2130
SG4044
0
10
20
30
40
Days after bacterial infection (dpi)

【Figure 9】

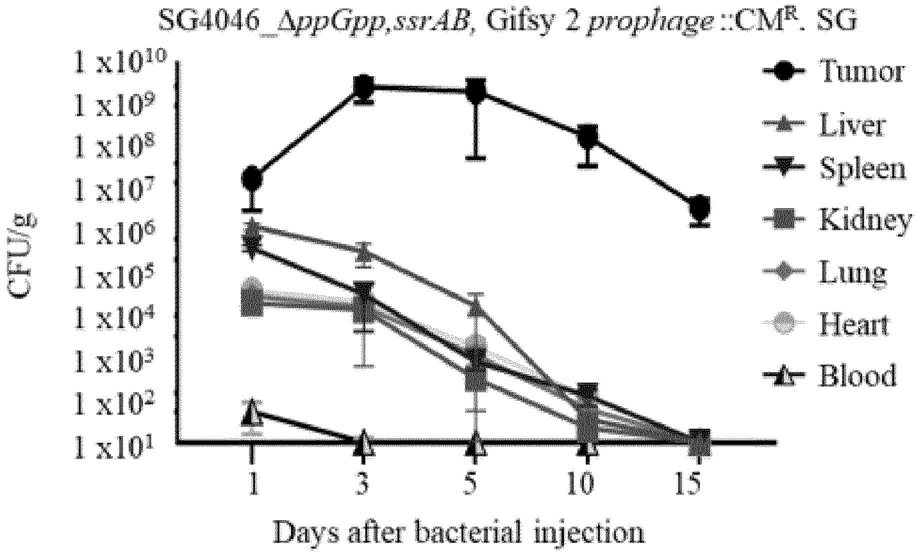
SG4046_ΔppGpp,ssrAB, Gifsy 2 prophage ::CMR. SG
CFU/g
1 x10^10
1 x10^9
1 x10^8
1 x10^7
1 x10^6
1 x10^5
1 x10^4
1 x10^3
1 x10^2
1 x10^1
1
3
5
10
15
Days after bacterial injection
Tumor
Liver
Spleen
Kidney
Lung
Heart
Blood

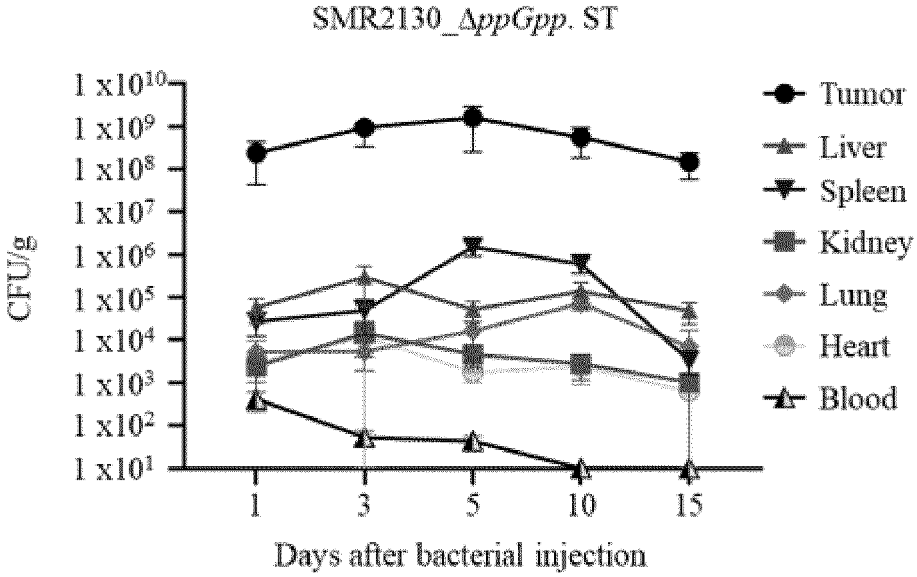
SMR2130_ΔppGpp. ST
CFU/g
1 x10^10
1 x10^9
1 x10^8
1 x10^7
1 x10^6
1 x10^5
1 x10^4
1 x10^3
1 x10^2
1 x10^1
1
3
5
10
15
Days after bacterial injection
Tumor
Liver
Spleen
Kidney
Lung
Heart
Blood

## INTERNATIONAL SEARCH REPORT

International application No.
**PCT/KR2021/005528**

**A. CLASSIFICATION OF SUBJECT MATTER**

**C12N 15/74**(2006.01)i; **C12N 9/12**(2006.01)i; **C12N 9/16**(2006.01)i; **A61K 39/112**(2006.01)i; **C12Q 1/02**(2006.01)i

According to International Patent Classification (IPC) or to both national classification and IPC

**B. FIELDS SEARCHED**

Minimum documentation searched (classification system followed by classification symbols)

C12N 15/74(2006.01); A61K 35/74(2006.01); A61K 39/02(2006.01); A61K 39/112(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 구아노신 4인산 합성효소(Guanosine tetraphosphate synthetase, ppGpp synthetase), 살모넬라 갈리나룸(Salmonella gallinarum), type III 분비 시스템(type III secretion system, T3SS), Gifsy 2 prophage, 약독화(attenuation), ssrA, ssrB, ssrAB, relA, spoT, glmS

**C. DOCUMENTS CONSIDERED TO BE RELEVANT**

| Category* | Citation of document, with indication, where appropriate, of the relevant passages | Relevant to claim No. |
|---|---|---|
| Y | KR 10-2011-0126227 A (INDUSTRY FOUNDATION OF CHONNAM NATIONAL UNIVERSITY) 23 November 2011 (2011-11-23)<br>See claims 1 and 2; paragraphs [0018] and [0078]; and table 1. | 1-15 |
| Y | RODWELL, E. V. et al. Isolation and characterisation of bacteriophages with activity against invasive non-typhoidal Salmonella causing bloodstream infection in Malawi. Viruses. 15 March 2021, vol. 13, thesis no. 478, pp. 1-17.<br>See page 2, second paragraph. | 1-15 |
| Y | KIM, K. et al. A novel balanced-lethal host-vector system based on glmS. PLoS One. 28 March 2013, vol. 8, no. 3, e60511 (pp. 1-11).<br>See abstract. | 2,6,8-11 |
| Y | KR 10-2014-0107954 A (INDUSTRY FOUNDATION OF CHONNAM NATIONAL UNIVERSITY) 05 September 2014 (2014-09-05)<br>See claims 1 and 4-7. | 12-15 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **25 January 2022** | **25 January 2022** |
| Name and mailing address of the ISA/KR<br>**Korean Intellectual Property Office**<br>**Government Complex-Daejeon Building 4, 189 Cheongsa-ro, Seo-gu, Daejeon 35208**<br>Facsimile No. **+82-42-481-8578** | Authorized officer<br><br>Telephone No. |

Form PCT/ISA/210 (second sheet) (July 2019)

INTERNATIONAL SEARCH REPORT

International application No.
**PCT/KR2021/005528**

**C. DOCUMENTS CONSIDERED TO BE RELEVANT**

| Category* | Citation of document, with indication, where appropriate, of the relevant passages | Relevant to claim No. |
|---|---|---|
| A | KR 10-1500805 B1 (CJ CHEILJEDANG CORPORATION) 09 March 2015 (2015-03-09)<br>See entire document. | 1-15 |

Form PCT/ISA/210 (second sheet) (July 2019)

**INTERNATIONAL SEARCH REPORT**

International application No.
**PCT/KR2021/005528**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   - a. ☑ forming part of the international application as filed:
     - ☑ in the form of an Annex C/ST.25 text file.
     - ☐ on paper or in the form of an image file.
   - b. ☐ furnished together with the international application under PCT Rule 13*ter*.1(a) for the purposes of international search only in the form of an Annex C/ST.25 text file.
   - c. ☐ furnished subsequent to the international filing date for the purposes of international search only:
     - ☐ in the form of an Annex C/ST.25 text file (Rule 13*ter*.1(a)).
     - ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (July 2019)

**INTERNATIONAL SEARCH REPORT**

International application No.
**PCT/KR2021/005528**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **16**
because they relate to subject matter not required to be searched by this Authority, namely:

Claim 16 pertains to a method for treatment of the human body by surgery, and thus pertains to a subject matter on which the International Searching Authority is not required to carry out an international search under the provisions of PCT Article 17(2)(a)(i) and PCT Rule 39.1(iv).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2019)

**INTERNATIONAL SEARCH REPORT**
**Information on patent family members**

International application No.
**PCT/KR2021/005528**

| Patent document cited in search report | Publication date (day/month/year) | Patent family member(s) | Publication date (day/month/year) |
|---|---|---|---|
| KR 10-2011-0126227 A | 23 November 2011 | None | |
| KR 10-2014-0107954 A | 05 September 2014 | KR 10-1548734 B1 | 01 September 2015 |
| KR 10-1500805 B1 | 09 March 2015 | BR 112013029677 A2 | 24 January 2017 |
| | | CL 2013003285 A1 | 13 June 2014 |
| | | CL 2017000232 A1 | 28 July 2017 |
| | | CN 104053768 A | 17 September 2014 |
| | | CN 104053768 B | 30 October 2020 |
| | | CN 107083351 A | 22 August 2017 |
| | | CN 107083351 B | 14 September 2021 |
| | | EP 2710116 A2 | 26 March 2014 |
| | | EP 2710116 B1 | 27 March 2019 |
| | | ES 2724729 T3 | 13 September 2019 |
| | | KR 10-2012-0128583 A | 27 November 2012 |
| | | PE 14522014 A1 | 23 October 2014 |
| | | PE 20141452 A1 | 23 October 2014 |
| | | US 2012-0294892 A1 | 22 November 2012 |
| | | US 2013-0022574 A1 | 24 January 2013 |
| | | US 2013-0202643 A1 | 08 August 2013 |
| | | US 8481052 B2 | 09 July 2013 |
| | | US 8524243 B2 | 03 September 2013 |
| | | US 9192658 B2 | 24 November 2015 |
| | | WO 2012-157989 A2 | 22 November 2012 |
| | | WO 2012-157989 A3 | 24 January 2013 |

Form PCT/ISA/210 (patent family annex) (July 2019)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- KR 102015573

### Non-patent literature cited in the description


- **FORBES, N.S.** Engineering the perfect (bacterial) cancer therapy. *Nature Reviews Cancer,* 2010, vol. 10 (11), 785-794
- **DATSENKO ; WANNER.** *Proceedings of the National Academy of Sciences,* 2000, vol. 97 (12), 6640-6645
- **TEDIN ; NOREL.** *J Bacteriol,* 2001, vol. 183, 6184-6196